# EUROPEAN PATENT APPLICATION

(11) **EP 2 116 295 A1**
(43) Date of publication of application: **11.11.2009**
(21) Application number: 08007451.1
(22) Date of filing: 16.04.2008
(51) Int. Cl.: B01J 8/00, C07C 29/151

(54) **Process for producing methanol from steam reforming**

(71) Applicant: METHANOL CASALE S.A., 6900 Lugano - Besso (CH)
(72) Inventor: Filippi, Ermanno, 6976 Castagnola (CH)
(74) Representative: Zardi, Marco

(57) **Abstract**

A process and plant for producing methanol from steam reforming, where a syngas (13) from steam reforming and having a certain hydrogen excess is mixed with a partially oxidized syngas (26) produced in a partial oxidation section (22) and having a low hydrogen content, thus obtaining a gaseous reactant with a balanced stoichiometric number in the high pressure synthesis loop (16). A revamping process for conventional steam reforming methanol plants is also disclosed, providing the addition of a partial oxidation section (22) in parallel to the existing reforming section (12).

## Description

### Field of the invention

The invention relates to a process and a plant for producing methanol from steam reforming of a hydrocarbon or hydrocarbon mixture. The invention is also useful in revamping existing steam-reforming methanol plants.

### Prior art

A large number of methanol plants in the world operate with a synthesis gas (syngas) produced by steam reforming of a hydrocarbon feedstock, usually a light hydrocarbon such as natural gas. The steam reforming process produces a reformed syngas mainly comprising carbon oxides (CO, CO₂) and hydrogen (H₂), which is then converted into methanol in a methanol high-pressure synthesis loop, also referred to as synthesis loop or HP loop. The term hydrocarbon feedstock is used with reference to a hydrocarbon or mixture thereof, for example natural gas mainly consisting of methane.

Referring more in detail to the steam reforming of a natural gas or substitute natural gas feedstock, the process of steam reforming can be summarized by the following reactions:

(I) CH₄ + H₂O → CO + 3H₂

(II) CO + H₂O → CO₂ + H₂

and reactions in the methanol HP synthesis loop can be summarized as:

(III) CO + 2H₂ → CH₃OH

(IV) CO₂ + 3H₂ → CH₃OH + H₂O

Reaction (I) of catalytic methane steam reforming is strongly endothermic, while the gas-shift reaction (II) is slightly exothermic, so that steam reforming for production of methanol requires an external heat supply. The steam reforming is usually carried out at about 850 °C and 25 bar pressure.

Conversion of the syngas to methanol, as known, is exothermic; it is also limited by the chemical equilibrium and unconverted reactants are recycled back to the reactor. The pressure in the HP loop is usually around 80 bar, that is greater than pressure in the reformer.

The main components of a steam-reforming methanol plant are a reformer fed with the natural gas and steam, and producing a syngas stream according to reactions (I) and (II), and the synthesis loop converting the syngas into raw methanol according to reactions (III) and (IV). A compressor is also necessary to raise the pressure of the syngas exiting the reformer to the high pressure of the HP loop.

The main components of the synthesis loop are usually a reactor, a pre-heater of the reagent gases, a condensation-separation section, and a circulation compressor. Raw methanol produced in the synthesis loop is sent to a distillation section, obtaining methanol with the desired degree of purity.

A recognized drawback of the above prior art is that the synthesis loop operates with a significant hydrogen excess. The hydrogen to carbon ratio (molar) is usually defined by the stoichiometric number SN according to the following definition:

SN = (H₂-CO₂) / (CO₂+CO)

In the rest of the description, the symbol SN will be used with reference to said stoichiometric number. The term hydrogen to carbon ratio is used, in a more general way, to indicate the ratio between number of moles of hydrogen and number of moles of carbon in the syngas. In this specification the term "carbon" is used, in a general way, to mean the content of carbon oxides CO and CO₂. The term "carbon oxides" is used to mean CO, CO₂ or a mixture thereof.

Optimum syngas to methanol conversion yield of reactions (III) and (IV) is achieved with SN around 2 - 2.1. The stoichiometric number of syngas from the steam reforming of natural gas, however, is close to 3, as can be understood from reactions (I) and (II). Hence, a syngas from steam reforming of natural gas has around 50% hydrogen excess, compared to the optimum hydrogen to carbon ratio for the methanol HP synthesis loop. Syngas from reforming of different light hydrocarbons may have less content of hydrogen but, in any case, hydrogen is in excess with respect to the above optimum value of SN ∼ 2.

Because of this hydrogen excess, the flow rate to be compressed and accommodated in the synthesis loop is larger than the flow rate that would be theoretically required for the given methanol output. In operation, the hydrogen in excess accumulates in the synthesis loop as unreacted material, still increasing the stoichiometric number for example up to SN = 14. This condition forces to a large oversizing of all the equipments of the HP synthesis loop. In other words, it may be stated that the available components of the synthesis loop, and especially the available volume of reaction, are not fully exploited by the known process.

This drawback is also felt in the field of revamping of conventional methanol plants. There are a large number of steam reforming methanol plants in the world which may benefit from an increase in the production capacity, which however is limited by the size and hence maximum flow rate permitted by the existing equipments such as vessels, piping, and so on, especially in the HP loop. With the known steam reforming process, an increase in the methanol output would require, in practical terms, to proportionally increase the flow rate circulated in the HP loop; in most cases this would involve replacement of the main and expensive components of the loop, making the revamping operation too costly.

### Summary of the invention

The problem underlying the invention is to increase the output of a steam reforming methanol plant in relation to the size of the equipments of the synthesis loop, that is to avoid the large over-sizing which is necessary in the prior art because of the hydrogen excess in the reactants.

The above problem is solved by a process for producing methanol comprising the steps of: steam reforming of a hydrocarbon feedstock obtaining a reformed syngas with a given hydrogen and carbon oxides content, and feeding said reformed syngas to a methanol synthesis loop, characterized by mixing said reformed syngas with a further gas stream containing hydrogen and carbon oxides, said further gas stream having a lower hydrogen to carbon ratio than said reformed syngas, obtaining a mixed gaseous reactant with a determined hydrogen to carbon ratio in said synthesis loop.

Referring to the stoichiometric number SN as defined above, and according to an aspect of the invention, a further gas stream with low SN is mixed with high-SN syngas from steam reforming, in a suitable proportion to obtain a gaseous mixed reactant with optimum stoichiometric number for conversion into methanol in said synthesis loop. Low and high SN are intended in relation to the optimum stoichiometric number around 2 - 2.1 for synthesis of methanol, i.e. the high-SN syngas has some hydrogen excess, while the low-SN syngas has some excess of carbon oxides.

Said further gas stream is preferably generated at a pressure greater than working pressure of the synthesis loop, so that it can be fed to the synthesis loop without being compressed.

According to one aspect of the invention, said further gas stream is a syngas obtained by partial oxidation of a hydrocarbon feedstock. The process of partial oxidation, in the most general way, can be carried out according to the following reaction:

(V) CHₓ + ½ O₂ → CO + x/2 H₂.

Preferably, the hydrocarbon feedstock used for generating the reformed syngas and the further syngas stream with low stoichiometric number is natural gas or substitute natural gas SNG. In a preferred application of the invention, a reformed syngas from steam reforming of natural gas or SNG, with a high stoichiometric number greater than optimum stoichiometric number for methanol conversion, is mixed with a further syngas obtained from steam reforming of natural gas or SNG, with addition of an oxygen stream, and having a low stoichiometric number less than said optimum stoichiometric number for methanol conversion, in a suitable proportion to obtain a gaseous mixed reactant with a balanced stoichiometric number in said synthesis loop.

More in detail, and according to preferred embodiments, a reformed syngas stream obtained from steam reforming of natural gas and having SN ∼ 3 is mixed with a further syngas stream obtained from partial oxidation of natural gas and having SN in the range 1.0 to 1.8, preferably around 1.4 - 1.6, in a suitable proportion to obtain a gaseous mixed reactant with stoichiometric number SN - 2 in said synthesis loop.

It should be noted that the high-SN gas from steam reforming and the low-SN gas can be mixed in any suitable point of the plant. Preferably, the high-SN gas from steam reforming and the low-SN gas from partial oxidation are both fed to the synthesis loop, where they mix forming a gaseous mixture with a balanced stoichiometric number.

In a preferred embodiment, said further gas stream is obtained in a suitable partial oxidation (POX) section operating at a pressure slightly above the pressure of the synthesis loop, in parallel to a steam reforming section producing said reformed syngas.

An object of the invention is also a plant adapted to operate with the above process. In particular, an object of the invention is a plant for producing methanol by steam reforming, comprising a reforming section to obtain a reformed syngas from steam reforming of a hydrocarbon feedstock, and a synthesis loop for converting said reformed syngas to methanol, characterized by further comprising a partial oxidation section operating in parallel to said reforming section, said partial oxidation section being fed with a hydrocarbon feedstock and providing a partially oxidized gas stream with a lower hydrogen to carbon ratio than said reformed syngas, the plant also providing a mixing of said partially oxidized gas stream with said reformed syngas.

The mixing of said partially oxidized gas stream with the reformed syngas is obtained for example with a flow line feeding the gas stream from the partial oxidation section to the synthesis loop, where it is mixed with the reformed gas coming from the reforming section. Said gas streams however can also be mixed together before entering the synthesis loop.

The partial oxidation section preferably operates at a pressure slightly above the pressure of the synthesis loop, that is significantly higher than pressure in the reformer. This requires to feed the natural gas to the partial oxidation section via a feeding compressor, but on the other hand avoids the need of a compressor between the partial oxidation section and the synthesis loop. Advantageously, the plant is also equipped with an air separation unit providing an O₂ stream to the partial oxidation section. In preferred embodiments, both the reforming section and the partial oxidation section operate with natural gas or SNG.

Another object of the invention is a method for revamping a steam reforming methanol plant, the methanol plant comprising a reforming section to obtain a syngas from steam reforming of a hydrocarbon feedstock, and a synthesis loop for converting said syngas to methanol, the method being characterized by:
- adding a partial oxidation section of a hydrocarbon in parallel to said reforming section, said partial oxidation section being adapted to provide a partially oxidized gas stream with a lower hydrogen to carbon ratio that the syngas from said steam reforming section;
- providing at least a flow line to mix said partially oxidized gas stream with the reformed syngas from the existing reforming section.

Preferably the added partial oxidation (POX) section is fed with the same hydrocarbon feedstock available for the reforming section, usually natural gas mainly consisting of methane. For example, the available feedstock is split into a first flow fed to the existing reforming section and a second flow fed to the new added POX section.

Preferably the added POX section operates at the pressure slightly above the pressure of the synthesis loop, so that a flow output line can connect the POX section to the synthesis loop, without the need to add a compressor between the new POX section and the existing synthesis loop.

According to another aspect of the revamping method, if the existing synthesis loop has adiabatic reactor(s), said reactors are shifted to isothermal operation, i.e. they are revamped to isothermal reactors or replaced with isothermal reactors, to cope with the higher concentration of reactants in the synthesis loop.

The term isothermal reactor in this specification is directed to a reactor equipped with an internal heat exchanger or heat exchange unit, preferably a plate heat exchanger, adapted to remove the heat output of the chemical reaction of methanol synthesis. The terms pseudo-isothermal operation and pseudo-isothermal reactors are also used with the same meaning.

Hence, the revamping of an existing adiabatic reactor involves the provision of an internal heat exchanger and related equipment, such as the piping of the cooling fluid, inside the existing vessel of the reactor; replacement of an existing adiabatic reactor with a new isothermal reactor however may be preferred according to the circumstances. Shift to the isothermal operation may be appropriate due to the fact that a higher concentration of reactants means more heat output of the exothermic reaction of methanol conversion.

The revamping intervention may include further modification of the existing plant, according to the needs. For example an intervention on a typical steam reforming plant may comprise the provision of the new POX section, the provision of an air separation unit (ASU) to feed oxygen to the POX section; the modification of the synthesis loop section from adiabatic to isothermal operation; revamping of the distillation section for adaptation to the larger raw methanol output of the synthesis loop.

The advantages of the invention are the following. The hydrogen excess in the reformed syngas is balanced by the further syngas from POX section, and an overall stoichiometric number close to the optimum value can be obtained in the synthesis loop. The resulting mixed syngas has a higher concentration of reactants CO and CO₂, and therefore it can reach a much higher conversion for each pass in the HP synthesis loop, allowing a higher production for a given circulation flow rate, in comparison with the prior art.

It can be stated that, due to invention, the equipments of the synthesis section are best exploited, avoiding the large circulation of unreactant H₂ which affects the prior-art steam reforming methanol plants.

The partial oxidation is preferred as it produces a gas with a high CO content, suitable to balance the hydrogen excess of the syngas from conventional steam reforming. A further advantage of the partial oxidation is that it can be carried out at a high pressure, i.e. eliminating the need of a compressor for the partially-oxidized syngas. To this purpose, the natural gas and oxygen fed to the POX unit must be compressed; this is however preferred to compression of the syngas because available compressors for natural gas and oxygen are much simpler and cheaper than a compressor specifically designed to work with a syngas. Moreover, compression of natural gas and O₂ requires less energy than compression of the syngas due to smaller molar flow. Feeding O₂ to the POX section gives the advantages of an overall thermally neutral reaction in said POX section, and lower hydrogen content of the produced gas.

The capacity of the HP synthesis loop, in terms of flow circulation, is used to circulate a balanced syngas with optimum stoichiometric content of CO, CO₂ and H₂, instead of a syngas containing a large quantity of H₂ in excess. This means that the production rate, in terms of raw methanol output compared to size of the equipment of the HP loop, is significantly increased over the prior art. Thank to the invention, a plant with a given size can achieve more methanol production or a given production can be obtained with smaller and less expensive equipments.

The advantages in the revamping intervention of an existing steam reforming methanol plant are that a much higher methanol output is obtained with few modification of the original synthesis loop. In fact, a conventional plant designed to operate with methane steam reforming is sized for a 50% hydrogen excess in the syngas, i.e. the original HP loop can easily accommodate an increased flow rate; the balanced gas obtained with the invention, on the other hand, is much more reactive, having more CO and CO₂ content and leaving less unreacted hydrogen in the loop. It should be appreciated that the equipments of the HP loop are quite expensive, due to the need to operate at pressure around 80 bar or more, hence the best exploitation of the flow circulating in the HP loop is an important advantage.

The invention allows to increase the production rate without having to proportionally increase the gas flow rate in the loop itself. As an example, a double methanol output can be achieved with only 15% increase in the flow rate circulating in the HP loop, i.e. a slight increase which normally does not oblige to replace all the equipments. In the prior art, a double of the production rate would almost always require to provide a new HP loop. It follows that the revamping is made more attractive from the technical and economical point of view.

Advantages of the invention will be more evident with the following detailed description of a preferred embodiment, presented as a non-limitative example.

### Brief description of the figures

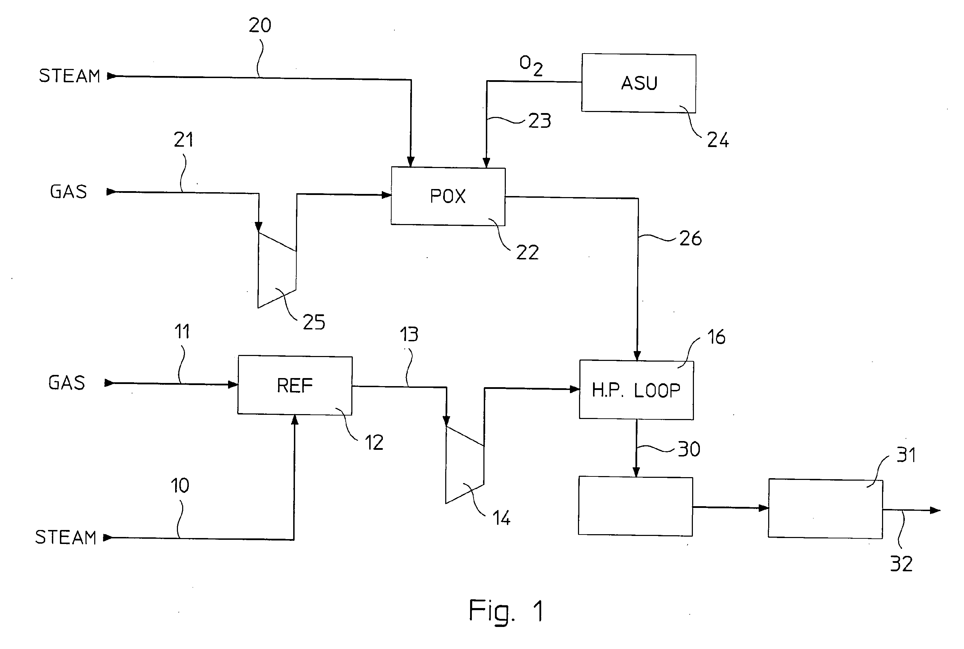

Fig. 1 is a simplified block diagram of a methanol plant operating according to the process of the invention, or a conventional steam reforming plant revamped according to the invention, with reference to a preferred embodiment.

### Detailed description of preferred embodiments

Referring to Fig. 1, a steam flow 10 and a natural gas flow 11 are fed to a reforming section 12, producing a reformed syngas 13 that after being cooled is fed to a high-pressure methanol synthesis loop 16 via a compressor 14. Said reforming section 12 and synthesis loop 16 are per se known and not described in detail.

The reforming section 12 comprises a reformer operating at about 850 °C and 25 bar pressure, with external heat supply (not shown) to furnish heat to the endothermic reaction (I) between methane and steam. The reformed syngas 13 produced in said section 12 has a stoichiometric number, as defined above, around SN=3.

A partial oxidation section or POX section 22 is installed in parallel to the reforming section 12, and receives a natural gas stream 21, a steam flow 20 and an oxygen flow 23 from an air separation unit (ASU) 24. Said POX section 22 provides a partially-oxidized POX syngas flow 26 with a low stoichiometric number, for example in the range 1.0 to 1.8 and preferably around 1.4 to 1.6, i.e. with a hydrogen to carbon ratio less than that of reformed syngas 13.

The POX section 22 operates at a high pressure, slightly above the pressure of the HP loop 16, so that the output of the POX section can be fed to said HP loop 16, via flow line 26, without additional compression. Pressure in the POX section 22, hence, is equal to pressure in the loop 16, plus at least the pressure loss through flow line 26. For example the pressure in the HP loop 16 can be around 80 bar and the pressure in the POX section 22 around 85 bar.

Usually the natural gas flows 11 directed to the reforming section 12, and the natural gas flow 21 directed to the POX section 22 are taken from the same feedstock. As the POX section 22 operates at a pressure greater than HP loop 16, i.e. well above the pressure of the reforming section 12, the pressure of the gas flow 21 directed to the POX section is raised in a suitable compressor 25.

The chemical reactions in POX section 22, fed with natural gas, can be summarized as:

2H₂ + O₂ → 2H₂O

CH4 + H2O → CO + 3H2

CO + H2O → CO2 + H2

The reaction between hydrogen and oxygen from ASU 24 helps to reduce the H₂ content of the gas, and is strongly exothermic providing the heat for the endothermic reaction between methane and steam. The above reactions are overall thermally neutral, so that no heat is to be exchanged. This means that the POX section 22 does not require a substantial heat supply or heat removal.

The POX syngas flow 26 is mixed in the synthesis loop 16 with the reformed syngas 13, thus balancing the overall stoichiometric value in said loop 16. Preferably the flow rates of streams 13 and 26 are determined to balance the overall stoichiometric value in the loop 16 at about SN - 2, namely close to the optimum value for synthesis of methanol. It can be appreciated that low SN of the POX syngas 26 compensates the high SN (excess of hydrogen) of the syngas 13, thus balancing the hydrogen/carbon content of the gas reacted in the synthesis loop 16.

The POX section 22 essentially comprises desuplhurizers for the natural gas 21, a POX reactor, a gas cooling train consisting for example of waste heat boilers, a gas-water washing column. The POX reactor of section 22 is for example a pressure vessel equipped with an oxygen injector for oxygen from the ASU unit 24.

The synthesis loop 16 yields a flow of raw methanol 30 which is sent to a distillation section 31, obtaining a grade AA methanol flow 32. The mixed gaseous reactant in the HP loop 16, resulting from mixing of reformed syngas flow 13 and POX syngas flow 26, is much more reactive than the reformed syngas 13, due to its balanced content of carbon oxides and hydrogen. This means that the mixed gaseous reactant achieves a higher output of methanol compared to the syngas 13 from prior-art reforming of natural gas.

It should be noted that the scheme of Fig. 1 is a simplified block diagram and components which are well known to the skilled man and not necessary for the understanding of the invention, as well as auxiliaries such as valves, etc... are not shown. For example, the stream 13 is normally cooled in a suitable gas cooler, not shown, before entering the compressor 14.

A revamping intervention according to a preferred implementation of the invention is now described.

A conventional steam-reforming methanol plant usually comprises at least the reforming section 12, fed with natural gas 11 and steam 10, and the synthesis loop 16 receiving the reformed syngas 13 produced in said reforming section 12. In the revamping operation, a new POX section 22 is added in parallel to the existing reforming section 12, and appropriate means are provided to feed the POX section with the natural gas stream 21, which can be taken from the same feedstock originating the gas stream 11.

The added POX section 22 is preferably designed to operate above the pressure of the existing HP synthesis loop 16. Accordingly, the compressor 25 is installed on the line of natural gas 21 directed to said POX section, to raise the pressure of the natural gas; in contrast, the output of the POX section can be fed without additional compression to the HP loop 16, i.e. there is no need of a gas compressor in line 26 between POX section 22 and loop 16.

An ASU unit 24 is also added to the existing plant, providing the oxygen stream 23 to the POX section 22. The ASU unit 24 can be provided as an autonomous package.

The new POX section 22 is designed to produce a POX syngas 26 with a hydrogen to carbon ratio (or stoiochiometric number) lower than that of syngas 13 produced by the existing reforming section. Said POX unit 22 is preferably sized so that the POX syngas 26, mixing with the reformed syngas 13 from the original equipment, gives an overall stoichiometric ratio around SN = 2. This means that the added POX section 22 is sized taking into consideration the flow rate and hydrogen content of the original reformed gas stream 13. Means are also provided to mix the syngas 26 with the syngas 13, for example providing a flow line from POX section 22 to the synthesis loop 16, so that the two gas streams are mixed in the high pressure loop forming a gaseous mixed reactant with balanced stoichiometric number.

A further step of the revamping method is the adaptation of the synthesis loop 16 to the higher concentration of reactants. In fact, the gas resulting from the mixing of streams 13 and 26 is much more reactive than the original stream 13, due to higher concentration of reactants (CO and CO₂), and less excess of hydrogen. The heat output of the methanol synthesis reaction is increased accordingly, and adaptation is necessary to avoid overheating of the reactor. Said adaptation involves in particular the internal equipments of the reactor(s) of the loop 16. If necessary, additional reactor(s) can be added.

In most cases, the existing reactor is an adiabatic reactor, which is commonly found in old steam reforming methanol plants. In this case, and in order to cope with the more reactive gas, the reactor is revamped to isothermal configuration, i.e. a suitable heat exchanger is installed inside the reactor, immersed in the catalytic bed, to remove the heat of reaction. Preferably said heat exchanger is a plate heat exchanger, but a tube heat exchanger can be used as alternative. Replacement of the existing reactor with a new isothermal reactor is also possible. Purely as an example, applicable isothermal reactor configurations are disclosed in EP 1284813 and WO 02/053276.

The distillation section can also be adapted to the larger methanol output of the revamped plant. As an example, the revamping may include new internals for an existing topping column and a new pre-heater, additional re-boiler and condenser, or the provision of a second HP refining column, with re-boiler and condenser. The existing compressor 14 feeding the natural gas to the synthesis loop can generally be maintained, as the flow 13 remains unchanged or is slightly increased.

The invention has been described with reference to steam reforming of natural gas or substitute natural gas; it is however applicable in a more general way to methanol conversion from steam reforming of hydrocarbons, especially light hydrocarbons ranging from natural gas to naphta.

## Claims

1. A process for producing methanol comprising the steps of steam reforming of a hydrocarbon feedstock (11) obtaining a reformed syngas (13) with a given hydrogen and carbon oxides content, and feeding said reformed syngas (13) to a methanol synthesis loop (16), **characterized by** mixing said reformed syngas (13) with a further gas stream (26) containing hydrogen and carbon oxides and having a lower hydrogen to carbon ratio that said reformed syngas (13), obtaining a mixed gaseous reactant with a determined hydrogen to carbon ratio in said synthesis loop (16).

2. A process according to claim 1, wherein said further gas stream (26) is generated at a pressure greater than the working pressure of said synthesis loop (16), so that said further gas stream (26) can be fed to the synthesis loop (16) without being compressed.

3. A process according to claim 1 or 2, wherein said further gas stream (26) is a syngas obtained by a process of partial oxidation of a hydrocarbon feedstock (21).

4. A process according to any one of preceding claims, wherein said reformed syngas (13) and said further gas stream (26) are obtained from natural gas or substitute natural gas SNG.

5. A process according to claim 4, wherein a reformed syngas stream (13) from steam reforming of natural gas or SNG, with a stoichiometric number greater than optimum stoichiometric number for methanol conversion, is mixed with a further syngas stream (26) obtained from steam reforming of natural gas or SNG with addition of an oxygen stream (23), and having a stoichiometric number less than said optimum stoichiometric number for methanol conversion, in a suitable proportion to obtain a gaseous mixed reactant with a balanced stoichiometric number in said synthesis loop (16), the stoichiometric number being defined as SN = (H₂-CO₂) / (CO₂+CO).

6. A process according to claim 5, wherein a reformed syngas stream (13) having SN - 3, is mixed with a further syngas stream (26) obtained from steam reforming of natural gas or SNG with addition of an oxygen stream (23), and having stoichiometric number SN in the range 1.0 to 1.8, in a suitable proportion to obtain a mixed gaseous reactant with overall SN - 2 in said synthesis loop (16).

7. A plant for producing methanol by steam reforming, comprising a reforming section (12) to obtain a reformed syngas (13) from steam reforming of a hydrocarbon feedstock (11), and a synthesis loop (16) for converting said reformed syngas (13) to methanol, **characterized by** further comprising a partial oxidation section (22) operating in parallel to said reforming section (12), said partial oxidation section (22) being fed with a hydrocarbon feedstock (21) and providing a partially oxidized gas stream (26) with a lower hydrogen to carbon ratio that said reformed syngas (13), the plant also providing a mixing of said partially oxidized gas stream (26) with said reformed syngas (13).

8. A plant according to claim 7, wherein said partial oxidation section (22) is adapted to operate at a pressure greater than the pressure of the synthesis loop (16).

9. A plant according to claim 8, further comprising a hydrocarbon feed (21) via a feeding compressor (25), a steam feed (20) and an air separation unit (24) providing an oxygen feed (23) to said partial oxidation section (22).

10. A method for revamping a steam reforming methanol plant, the methanol plant comprising a reforming section (12) to obtain a syngas (13) from steam reforming of a hydrocarbon feedstock (11), and a synthesis loop (16) for converting said syngas (13) to methanol, the method being **characterized by** the steps of:
- adding a partial oxidation section (22) of a hydrocarbon in parallel to said reforming section (12), said partial oxidation section being adapted to provide a partially oxidized gas stream (26) with a lower hydrogen to carbon ratio that the syngas (13) from said steam reforming section (12);
- providing at least a flow line to mix said partially oxidized gas stream (26) with the syngas (13) from the existing reforming section (12).

11. A method according to claim 10, wherein the added partial oxidation section (22) is adapted to operate at a pressure greater than the existing synthesis loop (16), and said flow line is adapted to feed the partially oxidized gas stream (26) from said partial oxidation section (22) to the synthesis loop (16) without intermediate compression.

12. A method according to claim 10 or 11, further comprising the provision of an air separation unit (24) feeding said partial oxidation section (22) with an oxygen stream (23).

13. A method according to any of claims 10 to 12, wherein adiabatic reactor(s) of the existing synthesis loop (16) are shifted to isothermal operation.

14. A method according to claim 13, wherein adiabatic reactor(s) of the existing synthesis loop (16) are revamped to isothermal operation by provision of an internal heat exchanger and related equipment in the original vessel of said reactor(s), or by replacing them with isothermal reactor(s) comprising an internal heat exchanger.
